# EUROPÄISCHE PATENTANMELDUNG

(11) **EP 0 670 311 A2**
(43) Veröffentlichungstag der Anmeldung: **06.09.1995**
(21) Anmeldenummer: 95102400.9
(22) Anmeldetag: 21.02.1995
(51) Int. Cl.: C07D 211/76, C07B 43/04

(54) **Verfahren zur Herstellung von Piperid-2-on**

(30) Priorität: 02.03.1994 DE 4406789
(71) Anmelder: BASF AKTIENGESELLSCHAFT, D-67056 Ludwigshafen (DE)
(72) Erfinder: Franz, Lothar, Dr., D-67112 Mutterstadt (DE)

(57) **Zusammenfassung**

Verfahren zur Herstellung von Piperid-2-on, indem man Polymere des delta-Valerolactons mit einer Molmasse von 200 bis 20.000 mit Ammoniak bei Temperaturen von 250 bis 500°C und Drücken von 30 bis 500 bar in Valerolacton oder in einem inerten Lösungsmittel in Abwesenheit eines Friedel-Crafts-Katalysators umsetzt.

## Beschreibung

Die vorliegende Erfindung betrifft ein Verfahren zur Herstellung von Piperid-2-on durch Umsetzung von Polymeren des delta-Valerolactons mit einer Molmasse von 200 bis 20.000 mit Ammoniak bei erhöhten Temperaturen und Drücken, das ohne einen Friedel-Crafts-Katalysator auskommt.

Aus Allgemeine und Praktische Chemie 618 bis 619 (1966), ist ein Verfahren zur Herstellung von Piperid-2-on durch Umsetzung von delta-Valerolacton mit Ammoniak bekannt.

Aus Liebigs Ann. Chem. 716, 83 bis 86 (1968), ist ein Verfahren zur Cyclisierung des Ammoniumsalzes der 5-Hydroxyvaleriansäure bekannt. Für diese Reaktion werden Temperaturen von über 300°C und Drücke von 100 bar benötigt.

Es ist allgemein bekannt, daß Valerolacton bereits durch geringe Wassermengen bei Raumtemperatur katalytisch polymerisiert wird und daher Wasser enthaltende Rohprodukte bereits vor der Destillation partiell polymerisieren.

Aus Chem. Abstr., Vol. 64, 8044b (1966), ist die Umsetzung von Polymeren des delta-Valerolactons zu Piperidon in Gegenwart von Friedel-Crafts-Katalysatoren bei 230 bis 300°C durch Aminierung bekannt. Geeignete Katalysatoren sind z.B. wasserfreie Chloride bzw. Bromide von Fe, Al, Zn, Sn oder Sb in einer Konzentration von bis zu 1%.

Nachteilig an dieser Vorgehensweise sind die verwendeten Katalysatoren, die sowohl Schwierigkeiten bei der Handhabung als auch Korrosionsprobleme verursachen können.

Der vorliegenden Erfindung lag daher die Aufgabe zugrunde, den zuvor genannten Nachteilen abzuhelfen.

Demgemäß wurde ein neues und verbessertes Verfahren zur Herstellung von Piperid-2-on gefunden, welches dadurch gekennzeichnet ist, daß man Polymere des delta-Valerolactons mit einer Molmasse von 200 bis 20.000 mit Ammoniak bei Temperaturen von 250 bis 500°C und Drücken von 30 bis 500 bar in Valerolacton oder in einem inerten Lösungsmittel in Abwesenheit eines Friedel-Crafts-Katalysators umsetzt.

Das erfindungsgemäße Verfahren kann wie folgt durchgeführt werden:
Eine Mischung bzw. Lösung aus Polymeren des delta-Valerolactons mit einer Molmasse von 200 bis 20.000, bevorzugt 500 bis 5.000, besonders bevorzugt 1.500 bis 4.000 (osmometrisch bestimmt) und ein Mischungs- bzw. Lösungsmittel kann bei Temperaturen von 250 bis 500°C, bevorzugt 270 bis 400°C, besonders bevorzugt 280 bis 330°C und Drücken von 30 bis 500 bar, bevorzugt 50 bis 400 bar, besonders bevorzugt 100 bis 300 bar in Abwesenheit eines Friedel-Crafts-Katalysators (Chem. Abstr., Vol. 64, 8044b (1966)), bevorzugt in Abwesenheit eines Aminierungskatalysators in Druckapparaturen wie Autoklaven und Röhrenreaktoren, unter Inertgasatmosphäre diskontinuierlich, bevorzugt kontinuierlich umgesetzt werden.

Als Inertgase eignen sich unter diesen Reaktionsbedingungen Wasserstoff, Stickstoff und Edelgas.

Als Mischungs- bzw. Lösungsmittel eignen sich Valerolacton, insbesondere delta-Valerolacton, oder unter den Reaktionsbedingungen inerte Lösungsmittel wie Pyridin, Triethylenglycol, Diethylenglycol, 1,2-Dimethoxyethan und Tetrahydrofuran, bevorzugt N,N'-Dimethylethylen- und N,N'-Dimethylpropylenharnstoff, besonders bevorzugt Lactame mit tertiärem Stickstoff wie N-Methylpyrrolidon, N-Octylpyrrolidon, 1-Dodecylpyrolidon und 1-(2-Ethylnexyl)pyrrolidon.

Das Piperid-2-on eignet sich als Zwischenprodukt für Pharma- und Pflanzenschutzwirkstoffe.

### Beispiele

### Beispiel 1

Zu einer Mischung von 198 g des festen Polymeren mit einer osmometrisch bestimmten Molmasse von 2310 und 99 g Valerolacton wurden im Autoklaven 600 ml Ammoniak gegeben und die Mischung 4 h bei 330°C unter einem Wasserstoffdruck von 280 bar umgesetzt. Nach der Destillation im Vakuum wurden 199 g (68 %) Piperidon erhalten.

### Beispiel 2

Zu einer Mischung von 236 g der festen Polymeren wie im Beispiel 1 und 197 N-Methylpyrrolidon wurden im Autoklaven 400 ml Ammoniak gegeben und die Mischung wie vor umgesetzt. Nach der Destillation im Vakuum erhielt man 195 g (83 %) Piperidon.

## Patentansprüche

1. Verfahren zur Herstellung von Piperid-2-on, dadurch gekennzeichnet, daß man Polymere des delta-Valerolactons mit einer Molmasse von 200 bis 20.000 mit Ammoniak bei Temperaturen von 250 bis 500°C und Drücken von 30 bis 500 bar in Valerolacton oder in einem inerten Lösungsmittel in Abwesenheit eines Friedel-Crafts-Katalysators umsetzt.

2. Verfahren zur Herstellung von Piperid-2-on nach Anspruch 1, dadurch gekennzeichnet, daß man Polymere des delta-Valerolactons mit einer Molmasse von 500 bis 5.000 einsetzt.

3. Verfahren zur Herstellung von Piperid-2-on nach Anspruch 1, dadurch gekennzeichnet, daß man die Umsetzung in Abwesenheit eines Aminierungskatalysators durchführt.

4. Verfahren zur Herstellung von Piperid-2-on nach Anspruch 1, dadurch gekennzeichnet, daß man als inerte Lösungsmittel N-Methylpyrrolidon einsetzt.
